# EUROPEAN PATENT APPLICATION

(11) **EP 3 533 446 A1**
(43) Date of publication of application: **04.09.2019**
(21) Application number: 17864656.8
(22) Date of filing: 30.10.2017
(51) Int. Cl.: A61K 31/375, A61K 31/185, A61K 31/07, A61K 31/047, A61K 36/82, A61P 27/02, A23L 33/155, A23L 33/15, A23L 33/105

(54) **COMPOSITION FOR PROTECTING EYESIGHT AND PREPARATION METHOD THEREFOR**

(30) Priority: 28.10.2016 CN 201610964273
(71) Applicant: Guangxi Sanpotel Health Industry Co. Ltd, Guangxi 530007 (CN)
(72) Inventor: HUANG, Aiqiang, Guangxi 530007 (CN); ZHONG, Wen, Guangxi 530007 (CN); HUANG, Aiyi, Guangxi 530007 (CN)
(74) Representative: Zaboliene, Reda
(86) International application number: PCT/CN2017/108390
(87) International publication number: WO 2018/077275

(57) **Abstract**

A composition for protecting eyesight, the composition including the following components in parts by weight: 30-100 parts of vitamin C sodium, 0.1-1 parts of vitamin A, 1-10 parts of lutein, 10-20 parts of taurine, and 10-30 parts of flower tea and fruit extract, the flower tea and fruit extract being prepared from the following raw ingredients in parts by weight: 40-60 parts of buddleja officinalis, 20-40 parts of camellia chrysantha, and 5-10 parts of fresh lemon. The composition has the effects of improving immunity, cooling, improving eyesight, and skin whitening, and can significantly improve symptoms such as blurred vision, dry eyes, eye swelling, eye pain, and photophobia.

## Description

### FIELD OF THE INVENTION

The present application belongs to the fields of medicines, health foods and foods, and relates to composition preparations and a preparation method thereof, in particular, to a composition for protecting eyesight and a preparation method thereof.

### BACKGROUND OF THE INVENTION

With the rapid development of modern science and technology and the popularization of the Internet, the use of computers has become more and more common. The number of Internet users in China has reached 710 million and the Internet penetration rate has reached 51.7%. As an mobile communication network environment is improving constantly and smart phones become more and more popular, the application of mobile Internet has penetrated into the life of Internet users, and the use rate of mobile Internet is also increasing day by day. At present, the number of mobile Internet users in China has reached 656 million. Radiation from computers and mobile phones is causing more and more damage to eyes, as a result, an increasing number of people have eye diseases now. In China, the number of myope is as high as 450 million, and the prevalence rate of myopia among young people ranks first in the world. With the growth of age, the prevalence rate of various eye diseases such as senile cataract and glaucoma is also increasing year by year. In order to address the problems of blurred vision, dry eyes, eye swelling, eye pain, photophobia and the like caused by the use of computers and mobile phones in daily life, so as to reduce the occurrence of eye diseases, a large number of eyesight protection products have appeared in the market, with efficacy ingredients being mainly chemical ingredients such as taurine, lutein and vitamins, but the effect is limited.

Vitamin C is a basic clinical drug or nutritional supplement and one of antioxidant vitamins. Vitamin C participates in the hydroxylation reaction in vivo, is necessary for the formation of intercellular adhesions of bones, teeth, connective tissues and non-epithelial tissues, and can maintain normal functions of teeth, bones and blood vessels and increase the resistance to diseases. Vitamin C is one of essential nutritional elements for human body and is widely used in the prevention and treatment of various diseases. The pH value of vitamin C aqueous solution is close to 2, the acidity is high, and oral administration will cause great irritation to oral cavity, throat, esophagus and gastric mucosa, so the vitamin C aqueous solution is not suitable for long-term administration, nor is the vitamin C aqueous solution suitable for simultaneous administration with acidic drugs. Therefore, its use is limited to a certain extent. Vitamin C sodium is the sodium salt of vitamin C, the pH value of the aqueous solution thereof is nearly neutral, and its function is the same as that of vitamin C. However, as a sodium salt, its performance is more stable; besides, vitamin C sodium no longer has the strong acidity of vitamin C, thus being able to be taken together with various drugs for a long term, so vitamin C sodium is better than vitamin C.

### SUMMARY OF THE INVENTION

In view of problems that existing eyesight protection products have limited effects, lozenges containing vitamin C are highly acidic after dissolution, can cause great irritation to oral cavity, throat, esophagus and gastric mucosa, and are not suitable for long-term administration, vitamin C tends to oxidize and lose efficacy in the storage process, and lozenges can easily absorb moisture and become damp, etc., the present application provides a composition for protecting eyesight and a preparation method thereof. The composition can also used for improving immunity, clearing heat, improving eyesight, whitening skin and the like. The present application solves the problems that similar products in the market have limited effects, vitamin C is unstable in quality during clinical application and not suitable for long-term administration and the like. The composition for protecting eyesight is safe, has no toxic or side effects, and is suitable for long-term use in addressing the problems of blurred vision, dry eyes, eye swelling, eye pain, photophobia and the like; or for long-term use as a nutritional supplement.

In order to solve the above technical problems, the composition for protecting eyesight and the preparation method thereof are realized through the following technical solution:
A composition for protecting eyesight includes the following components in parts by weight: 30-100 parts of vitamin C sodium, 0.1-1 part of vitamin A, 1-10 parts of lutein, 10-20 parts of taurine and 10-30 parts of flower tea and fruit extract.

The flower tea and fruit extract is prepared from the following raw ingredients in parts by weight: 40-60 parts of buddleja officinalis, 20-40 parts of camellia chrysantha and 5-10 parts of fresh lemon.

A preparation method of the flower tea and fruit extract includes:
S1: weighing the following raw ingredients in parts by weight: 40-60 parts of camellia chrysantha, 20-40 parts of camellia chrysantha and 5-10 parts of fresh lemon;
S2: adding 6-10 times of water to the weighed buddleja officinalis and camellia chrysantha, soaking for 30 minutes, extracting for 2-3 times, each time for 30-40 minutes, and filtering to obtain a flower tea extracting solution;
S3: cleaning and peeling the weighed fresh lemon, squeezing to obtain initial juice, and filtering to obtain lemon juice; and
S4: mixing the flower tea extracting solution with the lemon juice, concentrating under reduced pressure below 80°C to obtain a fluid extract with the relative density of 1.18-1.22 at 60°C, and drying the fluid extract at the drying temperature of 65-80°C to obtain the flower tea and fruit extract.

The extraction method in step S2 is preferably heating reflux extraction or ultrasonic extraction.

The drying method in step S4 is preferably oven heating drying or spray drying.

The composition for protecting eyesight described above preferably further includes the following components in parts by weight: 100-300 parts of starch, 200-500 parts of sucrose, 5-20 parts of mannitol, 10-50 parts of microcrystalline cellulose, 20-100 parts of sodium carboxymethyl starch, 5-20 parts of povidone K-30, 5-20 parts of sodium dodecyl sulfate, 1-5 parts of magnesium stearate and 0.1-0.5 part of mint essence.

The composition for protecting eyesight described above preferably includes the following components in parts by weight: 70 parts of vitamin C sodium, 0.5 part of vitamin A, 5 parts of lutein, 15 parts of taurine, 20 parts of flower tea and fruit extract, 200 parts of starch, 400 parts of sucrose, 10 parts of mannitol, 30 parts of microcrystalline cellulose, 60 parts of sodium carboxymethyl starch, 15 parts of povidone K-30, 10 parts of sodium dodecyl sulfate, 3 parts of magnesium stearate and 0.3 part of mint essence.

The flower tea and fruit extract is preferably prepared from the following raw ingredients in parts by weight: 50 parts of buddleja officinalis, 30 parts of camellia chrysantha and 8 parts of fresh lemon.

A preparation method of the composition for protecting eyesight includes the following steps:
T1: preparing the flower tea and fruit extract, weighing the following raw ingredients in parts by weight: 40-60 parts of buddleja officinalis , 20-40 parts of camellia chrysantha and 5-10 parts of fresh lemon; adding 6-10 times of water to the weighed buddleja officinalis and camellia chrysantha, soaking for 30 minutes, extracting for 2-3 times, each time for 30-40 minutes, and filtering to obtain a flower tea extracting solution; cleaning and peeling the weighed fresh lemon, squeezing to obtain initial juice, and filtering to obtain lemon juice; and mixing the flower tea extracting solution with the lemon juice, concentrating under reduced pressure below 80°C to obtain a fluid extract with the relative density of 1.18-1.22 at 60°C, and drying the fluid extract at the drying temperature of 65-80°C to obtain the flower tea and fruit extract;
T2: respectively taking vitamin C sodium, vitamin A, lutein, taurine, flower tea and fruit extract, starch, sucrose, mannitol, microcrystalline cellulose, sodium carboxymethyl starch, povidone K-30 and sodium dodecyl sulfate, pulverizing, and sieving with a 100-mesh sieve for later use;
T3: weighing the raw and auxiliary materials in step T2 according to the formula amount, and uniformly mixing by an equal increment method to obtain mixed powder;
T4: taking the mixed powder in step T3, adding 30-50% sucrose solution while stirring to prepare a damp mass, sieving and granulating, drying for 4 hours, controlling the moisture content to be 2-4%, and sieving and sizing to obtain granules; and
T5: weighing magnesium stearate and mint essence according to the formula amount, evenly mixing with the granules in step T4, and compressing the granules into lozenges.

The drying temperature in step T4 is preferably 60-65°C, so that the obtained granules have moderate hardness so as to be more favorable for tabletting. A screen used for sieving in step T4 is preferably a 14-30 mesh screen.

The present application provides has the beneficial effects:
1. The problems that existing lozenges containing vitamin C are highly acidic after dissolution, can cause great irritation to oral cavity, throat, esophagus and gastric mucosa, and are not suitable for long-term administration, vitamin C tends to oxidize and lose efficacy in the storage process, and lozenges can easily absorb moisture and become damp are solved, and the product has better stability and more exact curative effect, is suitable for long-term administration by consumers or patients, and has no side effects;
2. The raw ingredients of the flower tea and fruit extract in the present application are both medicinal and edible, where buddleja officinalis has the effects of dispelling wind, cooling blood, moistening liver and improving eyesight, and is used for treating red eye, eye swelling and pain, hyperdacryosis, photophobia, glaucoma and cataract, and red ulcerated eyelid, and is a special ophthalmic medicine; camellia chrysantha is a plant with high medicinal value and health care value, is unique to Guangxi and non-toxic, and contains more than 400 kinds of nutrients; studies show that camellia chrysantha has obvious effects on regulating blood lipid, blood sugar and cholesterol and enhancing immunity, and it has been used in folk for refreshing the brain, clearing liver fire, relieving fever and toxic substances, and nourishing vital energy; fresh lemon has the effects of whitening the skin, clearing away heat and phlegm, resisting bacteria and inflammation, preventing cardiovascular diseases and the like; and the combination of the three drugs has the effects of improving immunity, lowering blood sugar, blood pressure and blood lipid, clearing heat, improving eyesight, whitening skin and the like, so that the effects of treating blurred vision, dry eyes, eye swelling, eye pain, photophobia and the like are further strengthened, and the problem of single composition and function of eyesight protection products in the market is overcome; and
3. The composition for protecting eyesight of the present application is easy to prepare and good in granule fluidity, sticking does not occur during tabletting, and the prepared tablets have smooth surfaces, are not affected by dampness easily, taste sweet, have stable quality, are convenient to carry and eat, are especially suitable for children and the elderly, have high bioavailability, are safer and more effective to use, and are suitable for long-term use as a nutritional supplement.

### DETAILED DESCRIPTION OF THE INVENTION

Although the specification concludes with claims particularly pointing out and distinctly claiming the protection of the present application, it is believed that the following description will facilitate a better understanding of the present application.

As used herein, the word "preferred" and variants thereof refer to embodiments of the present application which are capable of providing specific benefits under specific circumstances. However, other embodiments may also be preferred under the same or other circumstances. Furthermore, the detailed description of one or more preferred embodiments does not indicate that other embodiments are useless and is not intended to exclude other embodiments from the scope of the present application.

### I. Selection of Preparation Conditions

### 1. Adhesive Selection

In the present application, dry tabletting, and wet granulation and tabletting are compared. And since dry tabletting has a high requirement for equipment and cannot generate a good effect by adopting a common tabletting machine, wet granulation and tabletting is adopted herein. In the experiment, 30% starch slurry, 40% sucrose syrup, 5% hydroxypropyl methylcellulose aqueous solution and 60% ethanol were compared as adhesives. Results are shown in Table 1.

**Table 1 Investigation Results of Wetting Agent**

| Serial number | Adhesive | Granulation condition | Granule appearance |
|---|---|---|---|
| 1 | 30% starch slurry | clustering, agglomerating easily during granulation | hard granules |
| 2 | 40% sucrose syrup | not clustering, not agglomerating during granulation | appropriate granule tightness |
| 3 | 5% hydroxypropyl methylcellulose aqueous solution | slightly clustering, agglomerating easily during granulation | relatively hard granules |
| 4 | 60% ethanol | not clustering, hard to granulate | loose granules |

From the test results in Table 1, it can be seen that 40% of sucrose used in the present application has the best granulation effect, so in the present application, the sucrose solution is selected as the adhesive. The present application continues to optimize the concentration of the sucrose solution, and the test results are shown in Table 2.

**Table 2 Investigation Results of Sucrose Solution Concentration**

| Serial number | Wetting agent | Granulation condition | Granule appearance |
|---|---|---|---|
| 1 | 20% sucrose solution | not clustering, hard to granulate | loose granules |
| 2 | 30% sucrose solution | clustering, not agglomerating during granulation | appropriate granule tightness |
| 3 | 40% sucrose solution | clustering, not agglomerating during granulation | appropriate granule tightness |
| 4 | 50% sucrose solution | clustering, not agglomerating during granulation | appropriate granule tightness |
| 5 | 60% sucrose solution | clustering, agglomerating during granulation | tight granules |

From the test results in Table 2, it can be seen that the 30%-50% sucrose solution used in the present application has a good granulation effect, tightness is too low for a lower concentration but too high for a higher concentration, which both affect granulation, so in the present application, the 30%-50% sucrose solution is selected as the adhesive.

### 2. Lubricant Selection

During tabletting, in order to increase granule fluidity, and ensure good filling and even distribution of tablet density, a certain amount of lubricant needs to be added to solve the problems of poor granule fluidity and sticking during tabletting. In the experiment of the present application, tabletting conditions when magnesium stearate, micro-powder silica gel, talcum powder, sodium dodecyl sulfate and mannitol were used as the lubricant were compared, and results are shown in Table 3.

**Table 3 Lubricant Trial Results**

| Lubricant | Fluidity | Tabletting condition | Tablet weight difference | Appearance | Disintegration time |
|---|---|---|---|---|---|
| mannitol | good fluidity | non-sticking | meeting specification | smooth and neat tablet surface, uniform color | meeting specification |
| micro-powder silica gel | good fluidity | sticking | meeting specification | smooth and neat tablet surface, nonuniform color | not meeting specification |
| talcum powder | poor fluidity | sticking | meeting specification | smooth and neat tablet surface, nonuniform color | not meeting specification |
| sodium dodecyl sulfate | good fluidity | non-sticking | meeting specification | smooth and neat tablet surface, uniform color | meeting specification |
| magnesium stearate | good fluidity | non-sticking | meeting specification | smooth and neat tablet surface, uniform color | meeting specification |

From the test results in Table 3, it can be seen that when magnesium stearate, sodium dodecyl sulfate and mannitol are added, fluidity is good and sticking during tabletting is avoided, and prepared tablets have appropriate hardness and smooth and beautiful surfaces; and when micro-powder silica gel and talcum powder are added, fluidity is poor, and sticking occurs during tabletting, prepared tablets have large hardness, and disintegration time does not meet specification. Therefore, magnesium stearate, sodium dodecyl sulfate and mannitol are selected as the lubricant in the present application. In order to adjust the taste of the chewable tablets and in consideration of production costs, in the experiment of the present application, the proportion of magnesium stearate to sodium dodecyl sulfate to mannitol was also compared, and the results show that the taste is the best when the proportion of magnesium stearate to sodium dodecyl sulfate to mannitol is 3:3:1 or 4:4:1.

### II. Preparation Method of Composition for Protecting Eyesight

### Embodiment 1

A preparation method of the composition for protecting eyesight includes the following steps:
T1: preparing the flower tea and fruit extract: weighing the following raw ingredients in parts by weight: 40 kg of buddleja officinalis, 20 kg of camellia chrysantha and 5 kg of fresh lemon; adding 6 times of water to the weighed buddleja officinalis and camellia chrysantha, soaking for 30 minutes, conducting ultrasonic extraction for 3 times, each time for 30 minutes, and filtering to obtain a flower tea extracting solution; cleaning and peeling the weighed fresh lemon, squeezing to obtain initial juice, and filtering to obtain lemon juice; and mixing the flower tea extracting solution with the lemon juice, concentrating under reduced pressure below 80°C to obtain a fluid extract with the relative density of 1.20 at 60°C, and conducting spray drying on the fluid extract at the drying temperature of 70°C to obtain the flower tea and fruit extract;
T2: respectively taking vitamin C sodium, vitamin A, lutein, taurine, flower tea and fruit extract, starch, sucrose, mannitol, microcrystalline cellulose, sodium carboxymethyl starch, povidone K-30 and sodium dodecyl sulfate, pulverizing, and sieving with a 100-mesh sieve for later use;
T3: weighing the following raw and auxiliary materials in step T2 in parts by weight: 30 kg of vitamin C sodium, 0.1 kg of vitamin A, 1 kg of lutein, 10 kg of taurine, 10 kg of flower tea and fruit extract, 100 kg of starch, 200 kg of sucrose, 5 kg of mannitol, 10 kg of microcrystalline cellulose, 20 kg of sodium carboxymethyl starch, 5 kg of povidone K-30 and 5 kg of sodium dodecyl sulfate, and uniformly mixing by an equal increment method to obtain mixed powder;
T4: taking the mixed powder in step T3, adding 30% sucrose solution while stirring to prepare a damp mass, sieving with a 20-mesh sieve and granulating, drying for 4 hours at 62°C, controlling the moisture content to be 2-4%, and sieving with a 20-mesh sieve and sizing to obtain granules; and
T5: weighing the following components in parts by weight: 1 kg of magnesium stearate and 0.1 kg of mint essence, evenly mixing with the granules in step T4, and compressing the granules into tablets.

### Embodiment 2

A preparation method of the composition for protecting eyesight includes the following steps:
T1: preparing the flower tea and fruit extract: weighing the following raw ingredients in parts by weight: 50 kg of buddleja officinalis , 30 kg of camellia chrysantha and 8 kg of fresh lemon; adding 8 times of water to the weighed buddleja officinalis and camellia chrysantha, soaking for 30 minutes, conducting heating reflux extraction twice, each time for 40 minutes, and filtering to obtain a flower tea extracting solution; cleaning and peeling the weighed fresh lemon, squeezing to obtain initial juice, and filtering to obtain lemon juice; and mixing the flower tea extracting solution with the lemon juice, concentrating under reduced pressure below 80°C to obtain a fluid extract with the relative density of 1.18 at 60°C, and conducting spray drying on the fluid extract at the drying temperature of 80°C to obtain the flower tea and fruit extract;
T2: respectively taking vitamin C sodium, vitamin A, lutein, taurine, flower tea and fruit extract, starch, sucrose, mannitol, microcrystalline cellulose, sodium carboxymethyl starch, povidone K-30 and sodium dodecyl sulfate, pulverizing, and sieving with a 100-mesh sieve for later use;
T3: weighing the following raw and auxiliary materials in step T2 in parts by weight: 70 kg of vitamin C sodium, 0.5 kg of vitamin A, 5 kg of lutein, 15 kg of taurine, 20 kg of flower tea and fruit extract, 200 kg of starch, 350 kg of sucrose, 10 kg of mannitol, 30 kg of microcrystalline cellulose, 60 kg of sodium carboxymethyl starch, 15 kg of povidone K-30 and 10 kg of sodium dodecyl sulfate, and uniformly mixing by an equal increment method to obtain mixed powder;
T4: taking the mixed powder in step T3, adding 40% sucrose solution while stirring to prepare a damp mass, sieving with a 30-mesh sieve and granulating, drying for 4 hours at 60°C, controlling the moisture content to be 2-4%, and sieving with a 30-mesh sieve and sizing to obtain granules; and
T5: weighing the following components in parts by weight: 3 kg of magnesium stearate and 0.3 kg of mint essence, evenly mixing with the granules in step T4, and compressing the granules into tablets.

### Embodiment 3

A preparation method of the composition for protecting eyesight includes the following steps:
T1: preparing the flower tea and fruit extract: weighing the following raw ingredients in parts by weight: 60 kg of buddleja officinalis , 40 kg of camellia chrysantha and 10 kg of fresh lemon; adding 10 times of water to the weighed buddleja officinalis and camellia chrysantha, soaking for 30 minutes, conducting ultrasonic extraction for 3 times, each time for 40 minutes, and filtering to obtain a flower tea extracting solution; cleaning and peeling the weighed fresh lemon, squeezing to obtain initial juice, and filtering to obtain lemon juice; and mixing the flower tea extracting solution with the lemon juice, concentrating under reduced pressure below 80°C to obtain a fluid extract with the relative density of 1.22 at 60°C, and drying the fluid extract in an oven at the drying temperature of 65°C to obtain the flower tea and fruit extract;
T2: respectively taking vitamin C sodium, vitamin A, lutein, taurine, flower tea and fruit extract, starch, sucrose, mannitol, microcrystalline cellulose, sodium carboxymethyl starch, povidone K-30 and sodium dodecyl sulfate, pulverizing, and sieving with a 100-mesh sieve for later use;
T3: weighing the following raw and auxiliary materials in step T2 in parts by weight: 100 kg of vitamin C sodium, 1 kg of vitamin A, 10 kg of lutein, 20 kg of taurine, 30 kg of flower tea and fruit extract, 300 kg of starch, 500 kg of sucrose, 20 kg of mannitol, 50 kg of microcrystalline cellulose, 100 kg of sodium carboxymethyl starch, 20 kg of povidone K-30 and 20 kg of sodium dodecyl sulfate, and uniformly mixing by an equal increment method to obtain mixed powder;
T4: taking the mixed powder in step T3, adding 70% sucrose solution while stirring to prepare a damp mass, sieving with a 14-mesh sieve and granulating, drying for 4 hours at 65°C, controlling the moisture content to be 2-4%, and sieving with a 14-mesh sieve and sizing to obtain granules; and
T5: weighing the following components in parts by weight: 5 kg of magnesium stearate and 0.5 kg of mint essence, evenly mixing with the granules in step T4, and compressing the granules into tablets.

### III. Stability Test

Samples from Embodiments 1-3 were packaged with double-layer aluminum-plastic composite films respectively, and placed in a stability acceleration test chamber for a three-month acceleration test, where the temperature is 40±-2°C, and the relative humidity is 75±5%. Results show that compared with test results of the samples at time 0, the samples from Embodiments 1-3 have no obvious changes in terms of key stability indexes such as appearance, moisture content, disintegration time, and main component content, indicating that the samples from Embodiments 1-3 of the present application are stable in quality and can meet stability requirements for storage, transportation, and use. The test results are shown in Table 4.

**Table 4 Stability Test Results of Lozenges for Protecting Eyesight**

| Embodiment | Time | Appearance | Moisture content (%) | Disintegration time | Vitamin C sodium content (by marked amount %) |
|---|---|---|---|---|---|
| Embodiment 1 | month 0 | yellow tablets | 2.24 | meeting specification | 101.02 |
| | month 1 | yellow tablets | 2.25 | meeting specification | 100.98 |
| | month 2 | yellow tablets | 2.25 | meeting specification | 100.94 |
| | month 3 | yellow tablets | 2.27 | meeting specification | 101.05 |
| Embodiment 2 | month 0 | yellow tablets | 2.36 | meeting specification | 99.58 |
| | month 1 | yellow tablets | 2.38 | meeting specification | 99.66 |
| | month 2 | yellow tablets | 2.38 | meeting specification | 99.71 |
| | month 3 | yellow tablets | 2.39 | meeting specification | 99.54 |
| Embodiment 3 | month 0 | yellow tablets | 2.31 | meeting specification | 98.97 |
| | month 1 | yellow tablets | 2.33 | meeting specification | 99.12 |
| | month 2 | yellow tablets | 2.34 | meeting specification | 98.92 |
| | month 3 | yellow tablets | 2.35 | meeting specification | 99.14 |

## Claims

1. A composition for protecting eyesight, **characterized by** comprising the following components in parts by weight: 30-100 parts of vitamin C sodium, 0.1-1 part of vitamin A, 1-10 parts of lutein, 10-20 parts of taurine and 10-30 parts of flower tea and fruit extract;
wherein the flower tea and fruit extract is prepared from the following raw ingredients in parts by weight: 40-60 parts of buddleja officinalis, 20-40 parts of camellia chrysantha and 5-10 parts of fresh lemon.

2. The composition for protecting eyesight according to claim 1, **characterized in that** a preparation method of the flower tea and fruit extract comprises:
S1: weighing the following raw ingredients in parts by weight: 40-60 parts of buddleja officinalis, 20-40 parts of camellia chrysantha and 5-10 parts of fresh lemon;
S2: adding 6-10 times of water to the weighed buddleja officinalis and camellia chrysantha, soaking for 30 minutes, extracting for 2-3 times, each time for 30-40 minutes, and filtering to obtain a flower tea extracting solution;
S3: cleaning and peeling the weighed fresh lemon, squeezing to obtain initial juice, and filtering to obtain lemon juice; and
S4: mixing the flower tea extracting solution with the lemon juice, concentrating under reduced pressure below 80°C to obtain a fluid extract with the relative density of 1.18-1.22 at 60°C, and drying the fluid extract at the drying temperature of 65-80°C to obtain the flower tea and fruit extract.

3. The composition for protecting eyesight according to claim 2, **characterized in that** the extraction method in step S2 is heating reflux extraction or ultrasonic extraction.

4. The composition for protecting eyesight according to claim 2, **characterized in that** the drying method in step S4 is oven heating drying or spray drying.

5. The composition for protecting eyesight according to any one of claims 2 to 4, **characterized in that** auxiliary materials are added to manufacture a preparation allowed in health foods and foods.

6. The composition for protecting eyesight according to claim 5, **characterized in that** the preparation is granule, pill, tablet, capsule, oral liquid, syrup, beverage or tea.

7. The composition for protecting eyesight according to claim 6, **characterized in that** the tablet is lozenge.

8. A lozenge prepared from the composition for protecting eyesight according to claim 7, **characterized by** comprising the following components in parts by weight: 100-300 parts of starch, 200-500 parts of sucrose, 5-20 parts of mannitol, 10-50 parts of microcrystalline cellulose, 20-100 parts of sodium carboxymethyl starch, 5-20 parts of povidone K-30, 5-20 parts of sodium dodecyl sulfate, 1-5 parts of magnesium stearate and 0.1-0.5 part of mint essence.

9. A method for preparing a lozenge from the composition for protecting eyesight according to claim 7, **characterized by** comprising the following steps:
T1: preparing a flower tea and fruit extract: weighing the following raw ingredients in parts by weight: 40-60 parts of buddleja officinalis, 20-40 parts of camellia chrysantha and 5-10 parts of fresh lemon; adding 6-10 times of water to the weighed buddleja officinalis and camellia chrysantha, soaking for 30 minutes, extracting for 2-3 times, each time for 30-40 minutes, and filtering to obtain a flower tea extracting solution; cleaning and peeling the weighed fresh lemon, squeezing to obtain initial juice, and filtering to obtain lemon juice; and mixing the flower tea extracting solution with the lemon juice, concentrating under reduced pressure below 80°C to obtain a fluid extract with the relative density of 1.18-1.22 at 60°C, and drying the fluid extract at the drying temperature of 65-80°C to obtain the flower tea and fruit extract;
T2: respectively taking vitamin C sodium, vitamin A, lutein, taurine, flower tea and fruit extract, starch, sucrose, mannitol, microcrystalline cellulose, sodium carboxymethyl starch, povidone K-30 and sodium dodecyl sulfate, pulverizing, and sieving with a 100-mesh sieve for later use;
T3: weighing the raw and auxiliary materials in step T2 according to the formula amount, and uniformly mixing by an equal increment method to obtain mixed powder;
T4: taking the mixed powder in step T3, adding 30-50% sucrose solution while stirring to prepare a damp mass, sieving and granulating, drying for 4 hours, controlling the moisture content to be 2-4%, and sieving and sizing to obtain granules; and
T5: weighing magnesium stearate and mint essence according to the formula amount, evenly mixing with the granules in step T4, and compressing the granules into lozenges.

10. The method for preparing a lozenge from the composition for protecting eyesight according to claim 9, **characterized in that** the drying temperature in step T4 is 60-65°C.

11. The method for preparing a lozenge from the composition for protecting eyesight according to claim 9, **characterized in that** a screen used for sieving in step T4 is a 14-30 mesh screen.
